Europäisches Patentamt

European Patent Office

Office européen des brevets

Publication number: **0 276 376**
**A2**

## EUROPEAN PATENT APPLICATION

(21) Application number: 87115802.8

(22) Date of filing: 28.10.87

(51) Int. Cl.⁴ **A61M 1/16**

(30) Priority: 29.01.87 SE 8700344

(43) Date of publication of application:
**03.08.88 Bulletin 88/31**

(84) Designated Contracting States:
**BE CH DE ES FR GB IT LI NL**

(71) Applicant: **GAMBRO AB**
**Post Box 10101**
**S-220 10 Lund(SE)**

(72) Inventor: **Knutsson, Stefan Lars**
**Fenix väg 6**
**S-237 00 Bjärred(SE)**
Inventor: **Shaldon, Stanley**
**Villa 86 Rue de Grézac**
**F-34100 Montpellier(FR)**

(74) Representative: **Boberg, Nils Gunnar Erik**
**Gambro AB Patent Department Box 10101**
**S-220 10 Lund(SE)**

(54) **A system for the preparation of a fluid intended for a medical treatment.**

(57) A system for the preparation of a fluid intended for a medical treatment by mixing of pure water with at least one concentrate, the concentrate being filtered before the mixing with the help of a filter (15), comprising a concentrate inlet (40) and an outlet (44) for the filtered concentrate and a connection (41), preferably completely closed during normal use, arranged on the inlet side (16) of the filter.

The system in accordance with the invention is characterized in that the filter (15) for the purpose of disinfection and/or sterilization or other cleaning is adapted to be flushed through on its inlet side (16) with a fluid intended for this purpose, a part of which is arranged to be withdrawn via the filtering material through the said outlet (44) for filtered concentrate.

The system in accordance with the invention is intended in particular for the preparation of dialysis fluid intended for haemodialysis. It may also be used, however, for the preparation of replacement fluid in connection with haemodiafiltration or haemofiltration.

Fig.1

# A SYSTEM FOR THE PREPARATION OF A FLUID INTENDED FOR MEDICAL TREATMENT

## TECHNICAL FIELD

The present invention relates to a system for the preparation of a fluid intended for a medical treatment by mixing of water with at least one concentrate, the concentrate being filtered before the mixing with the help of a filter comprising a concentrate inlet and an outlet for the filtered concentrate and a connection, preferably completely closed during normal use, arranged on the inlet side of the filter, that is to say on the same side of the filtering material as the concentrate inlet.

The system is intended in particular for the preparation of dialysis fluid in connection with haemodialysis. With minor modifications, however, it may also be used for the preparation of replacement fluid in connection with haemofiltration or haemodiafiltration. To those versed in the art it will be obvious too that the system can be used in connection with other methods of treatment where the mixing of pure water with at least one concentrate is required so as to produce a preferably wholly bacteria-free and substantially pyrogen-free solution.

In haemodialysis blood is conducted to the one side of the membrane in a dialyser, at the same time as dialysis fluid is conducted to the opposite side of the same membrane. The poisons, which one wishes to remove are passed in this case with the help of diffusion from the blood to the dialysis fluid. Normally a certain amount of fluid, primarily water, is withdrawn at the same time through ultrafiltration, so that a lowering of weight of the patient is brought about.

Haemodiafiltration differs from haemodialysis first and foremost in that a more permeable filter is used and consequently, greater ultrafiltration is obtained, which makes it necessary for a part of the ultrafiltrate to be replaced by a replacement fluid.

In heamofiltration no dialysis fluid is used. Instead, with the help of a filter, a larger quantity of ultrafiltrate is withdrawn which has to be at least partly replaced by a corresponding quantity of replacement fluid.

## BACKGROUND ART

For haemodialysis, haemodiafiltration and haemofiltration respectively different types of control arrangements are normally used which have in common that at least one concentrate is mixed with pure water. Certain such concentrates are of the type which prevents bacteria growth. No problems exist then, and the concentrate can be mixed directly with the water. Other concentrates, e g those based on bicarbonate, on the other hand, favour bacteria growth and have to be filtered, therefore, before they are mixed with the water. The filter used for this purpose are designed for low percolation which renders the sterilization and/or disinfection required between respective treatments difficult.

## DISCLOSURE OF INVENTION

It is an object of the present invention to eliminate the above-mentioned difficulties in connection with sterilization and/or disinfection of firstly the concentrate filter, and secondly the remainder of the system.

The above-mentioned problem is solved in that the filter, for the purpose of disinfection and/or sterilization or other cleaning is adapted to be flushed through on its inlet side with a fluid intended for this purpose, a part of which is arranged to be withdrawn via the filtering material through the said outlet for the filtered concentrate. This through flushing can be carried out irrespectively of the flows which normally occure through the filter, at the same time as its filtrate side still can be effectively treated.

The invention is thus intended to be applied in connection with sterilization as well as disinfection. However, for the sake of simplicity it will be described in the following only in connection with sterilization.

The said fluid is preferably arranged to be supplied via the concentrate inlet of the filter and is discharged to a drain via the said connection, which here is joined appropriately to a drain via a pressure control valve. As a result an appropriate through flushing of the inlet side of the filter can be achieved, at the saime time as a part of the fluid can be forced through the filtering material for an effective sterilization of the filtrate side of the filter.

At least a part of the fluid forced through the filtering material appropriately is passed further through the system with the help of a suction pump, e g one as normally used for the suction of concentrate through the same duct.

The system in accordance with the invention can also be applied to an installation comprising two sources of concentrate which are connected to the remaining system via two connecting ducts, whereof at least one is provided with a filter of the said type and each of which is connected to a suction pump. A simple change-over from normal treatment to sterilization can be achieved here if the two sources of concentrate are adapted so that they can be disconnected, with the two connecting ducts being joined together.

To improve in this case the percolation also of the filtrate side of the filter a branch duct connected to a drain may be adapted so as to originate from a point in the associated connecting duct between the said outlet for the filtered concentrate and the suction pump arranged in the same duct.

For the control of the flow through this branch duct a pressure control valve is suitably arranged. With the help of this and the above-mentioned pressure control valve appropriate flows can be achieved on the inlet side of the filter as well as on its filtrate side. The main principle in this context is that the pressure control valve between the said connection and the said outlet is set, or is adapted so that it can be set, to a higher resistance pressure than the pressure control valve in the said branch duct. Owing to this pressure difference a suitable amount of sterilizing fluid thus can be forced through the filtering material.

The water used must also be filtered prior to mixing with the help of a filter comprising a water inlet connected to a source of water, an outlet for filtered water and and outlet for non-filtered water connected to a drain. In a system of this type the said outlet for the filtered water in accordance with the invention is arranged so that it can be connected on the one hand to a point for mixing with the said concentrate, on the other hand directly to the concentrate filter. The supply duct for sterilizing fluid here may be arranged before the inlet of the water filter.

The direct connection between the water filter and the concentrate filter is used only in connection with sterilization and/or the subsequent rinsing. In consequence of this a shut-off valve has to be provided between the two filters.

BRIEF DESCRIPTION OF THE DRAWINGS

The invention is described in more detail in the following with reference to the attached drawings.

Fig 1 shows a system in accordance with the invention set up for haemodialysis.

Fig 2 shows the same system set up for chemical sterilization and/or the subsequent rinsing.

BEST MODE OF CARRYING OUT THE INVENTION

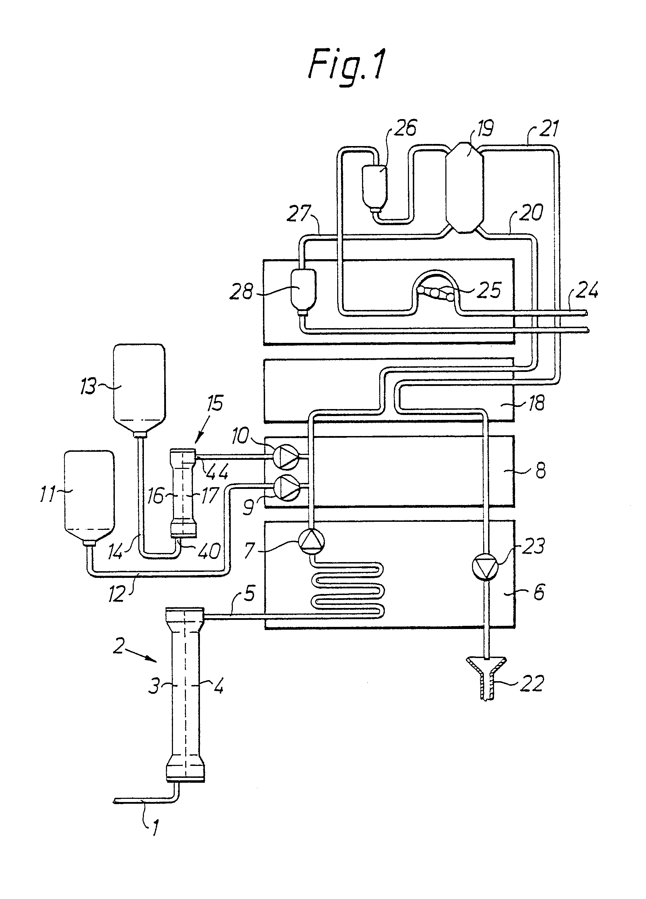

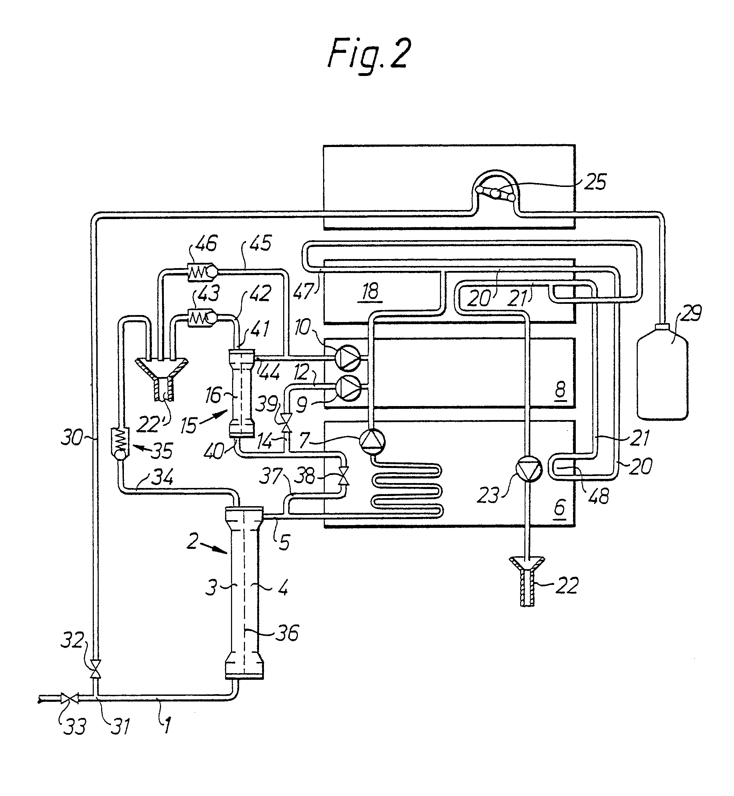

In fig 1 are shown primarily those components which are necessary for the actual dialysis treatment. The additional components for sterilization and/or rinsing will be found in fig 2. In both figures any components which are of less importance for an understanding of the invention, such as conventional pressure gauges, conductivity meters, temperature measuring instruments etc, have been left out.

In the set-up according to fig 1 water is supplied from a water source, not shown, via a duct 1 into a water filter 2 which is divided into an inlet side 3 and a filtrate side 4. The filtered water is passed via a duct 5 into a conventional dialysis monitor 6 with the necessary means for treatment of the water, e g heating and temperature and pressure checking. This monitor may be designed, for example, in accordance with US patent 4 158 034.

With the help of a pump 7 the water is conducted further to a mixing monitor 8 which in the case shown comprises two concentrate pumps 9 and 10 respectively. In normal dialysis treatmeant the concentrate pump 9 draws concentrate from a source 11 via a duct 12. In the same manner the pump 10 draws concentrate from a source 13 via a duct 14 and a filter 15. The filter 15 too can be said to be divided into an inlet side 16 and a filtrate side 17 respectively. After the mixing of the respective concentrate with the water the conductivity of the mixture is checked in a conductivity meter, not shown, which may be located in the conventional dialysis monitor 6 or in the mixing monitor 8. Further details regarding this mixing may be found in EP 0 022 922 B1.

From the mixing monitor 8 the dialysis solution prepared is passed via the flow control monitor 18 to a dialyser 19 with the help of a duct 20. From the dialyser 19 the dialysate obtained is passed back again via the flow control monitor 18 and the dialysis monitor 6 to a drain 22. This is done with the help of a suction pump 23 in order to maintain an appropriate low pressure in the said dialyser 19. The said flow control monitor 18 is described in more detail for example in US patent 4 585 552.

Blood is withdrawn from a patient (not shown) via a duct 24 and is pumped with the help of pump 25 via a drip chamber 26 to the dialyser 19 and from here via a duct 27 with a drip chamber 28 back again to the patient.

The system described above can be said to be largely conventional, and difficulties may arise therefore in connection with sterilization of the concentrate filter 15. However, the system can be modified in accordance with the invention in such a manner as shown in fig 2 wherein the same reference designations have been used as in fig 1 for components corresponding to one another. Thus the system is shown here modified for chemical sterilization and/or subsequent rinsing. As can be seen, the dialyser 19 and the associated blood ducts have been completely cut out. Instead the blood pump 25 is used for the supply of a sterilizing fluid, e g formaldehyde, from a source 29. This fluid is passed via a duct 30 to a point 31 on the water inlet duct. Designation 32 indicates - schematically either a shut-off cock or a disconnecting device. In the same manner designation 33 indicates a shut-off cock in the water duct 1. With the cock 32 open and the cock 33 closed sterilizing fluid can be introduced therefore on the inlet side 3 of the water filter 2, from where a part is conducted further via a duct 34 with a check valve 35 to a drain 22' which in principle may be identical with that designated 22. Alternatively concentrated sterilizing fluid is passed from the source 29 to the point 31, here to be mixed with an appropirate amount of water. Owing to the check valve 35, a part of the sterilizing fluid is forced to pass the filtering material which normally is constituted of one or more membranes 36 in the form of flat films or hollow fibres. This percolation is facilitated also by the pump 7 provided in the dialysis monitor 6. A part of the sterilizing fluid, however, is conducted via a duct 37 and a valve 38 directly to the concentrate filter 15. Just before the latter, though, a further partial stream is diverted through the connecting ducts 12 and 14 which are joined together at 39. This partial stream is drawn via the pump 9 into the main stream. The sterilizing fluid flows into the filter 15 via the normal concentrate inlet 40 and flushes through the inlet side 16 of this filter. It leaves the filter via a connection 41 and a duct 42 with a pressure control valve 43, ultimately to flow out into the drain 22'. Thanks to the pressure control valve 43, however, a part of the sterilizing fluid is pressed though the filtering material to the normal outlet 44 for filtered concentrate. The fluid flowing out through this outlet is divided once again into two partial streams, namely one via the pump 10 to the main duct and a second stream via a branch duct 45 with a pressure control valve 46 to the drain 22'.

In order to achieve an appropriate division of flow between the ducts 42 and 45 the pressure control valve 43 ought to be set, or be settable, to a higher resistance pressure than the pressure control valve 46. By way of example it can be said that at a pressure of 0.5-1.0 bar in the duct 5 it has been found appropriate to set the valve 43 to a counterpressure of approx. 300 mmHg and the valve 46 to a counterpressure of approx. 100 mmHg. At these pressures the valves 43 and 46 are shut automatically in normal operation.

From the pumps 7, 9 and 10 the sterilizing fluid flow further up into the flow control monitor 18 where it divides into a branch duct 47 and the duct 20 normally leading up to the dialyser. The branch duct 47 is intended to allow the system shown to be modified, so that it can be used also for haemofiltration and haemodiafiltration respectively. The way this is done is described in more detail in Swedish patent application 86.03746-2.

The duct 20 has been disconnected from the dialyser and is connected instead to a so-called bypass arrangement 48. The same applies to the duct 21 normally leading away from the dialyser. The manner this bypass arrangement can function is described. for example, in US patent 4 122 010. From the bypass arrangement the sterilizing fluid is conducted via the duct 21, the flow control monitor 18 and the dialysis monitor 6 with its pump 23 to the drain 22.

With regard to the filters 2 and 15, finally, it should be added that they ought to be bacteria-tight. By this is meant normally that they should allow not more than one bacterium per million inflowing bacteria to pass through. Moreover, pyrogens ought to be separated to a high degree. It is normally required in this respect that they should not allow more than one pyrogen particle per 10 000 inflowing particles of this kind to pass through.

Naturally the invention is not limited merely to the embodiment described above, but can be varied within the scope of the following claims. For example, the components included in the system can be varied within wide limits both with regard to their form and their function.

## Claims

1. A system for the preparation of a fluid intended for medical treatment by mixing of water with at least one concentrate, the concentrate being filtered before the mixing with the help of a filter (15) comprising a concentrate inlet (40) and an outlet (44) for the filtered concentrate and a connection (41), preferably completely closed during normal use, arranged on the inlet side (16) of the filter, **characterized** in that the filter (15) for the purpose of disinfection and/or sterilization or other cleaning is adapted to be flushed through on its inlet side (16) with a fluid intended for this purpose, a part of which is arranged to be withdrawn via the filtering material through the said outlet (44) for filtered concentrate.

2. A system in accordance with claim 1, **characterized** in that the said fluid is adapted to be supplied via the concentrate inlet (40) of the filter and to be discharged to a drain (22') via the said connection (41).

3. A system in accordance with claim 2, **characterized** by a pressure control valve (43) arranged between the said connection (41) and the said drain (22').

4. A system in accordance with anyone of the preceding claims, **characterized** by a suction pump (10) arranged after the said outlet (44) for filtered concentrate.

5. A system in accordance with anyone of the preceding claims comprising two sources fo concentrate (11,13) which are connected to the remaining system via two connecting ducts (12,14), whereof at least one (14) is provided with a filter (15) of the said type and each of which is connected to a suction pump (9 and 10 respectively), **characterized** in that the two sources of concentrate (11,13) are adapted so that they can be disconnected, with the two connecting ducts (12,14) being joined together.

6. A system in accordance with claim 5, **characterized** by a branch duct (45) connected to a drain (22') originating from a point in the associated connecting duct between the said outlet (44) for filtered concentrate and the suction pump (10) arranged in the same duct.

7. A system in accordance with claim 6, **characterized** by a pressure control valve (46) arranged in the said branch duct (45).

8. A system in accordance with claims 3 and 6, **characterized** in that the pressure control valve (43) between the said connection (41) and the said drain (22') is set, or is adapted so that it can be set, to a higher resistance pressure than the pressure control valve (46) in the said branch duct (45).

9. A system in accordance with anyone of the preceding claims, the water being filtered prior to mixing with the help of a filter (2) comprising a water inlet (1) connected to a source of water, an outlet (5) for filtered water and an outlet (34) for non-filtered water connected to a drain, **characterized** in that the said outlet (5) for the filtered water is adapted so that it can be connected on the one hand to a point for mixing with the said concentrate, on the other hand directly to the concentrate filter (15).

10. A system in accordance with claim 9, **characterized** by a supply duct (30) for the said fluid arranged before the inlet (1) of the water filter (2).

11. A system in accordance with claim 9 or 10, **characterized** by a shut-off valve (38) arranged between the water filter (2) and the concentrate filter (15).

# Fig.1

# Fig.2